(19) Europäisches Patentamt
European Patent Office
Office européen des brevets

(11) Veröffentlichungsnummer: 0 314 624
A1

# (12) EUROPÄISCHE PATENTANMELDUNG

(21) Anmeldenummer: **88810721.6**

(22) Anmeldetag: **21.10.88**

(51) Int. Cl.4: **C 07 D 498/18**
A 61 K 31/495
//(C07D498/18,323:00,307:00, 263:00)

(30) Priorität: **27.10.87 GB 8725118**
**30.06.88 CH 2500/88**

(43) Veröffentlichungstag der Anmeldung:
**03.05.89 Patentblatt 89/18**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Kump, Wilhelm, Dr.**
**Friedrich-Oser-Strasse 10**
**CH-4105 Biel-Benken (CH)**

**Menear, Keith Allan, Dr.**
**27 Stoneybrook Hills Farm Lane**
**Horsham West Sussex (GB)**

Patentansprüche für folgende Vertragsstaaten: ES + GR.

(54) **Substituierte Azacyclohexyl-Derivate.**

(57) Die Erfindung betrifft die Herstellung von substituierten Azacyclohexyl-Derivaten von Rifamycinen der Formel

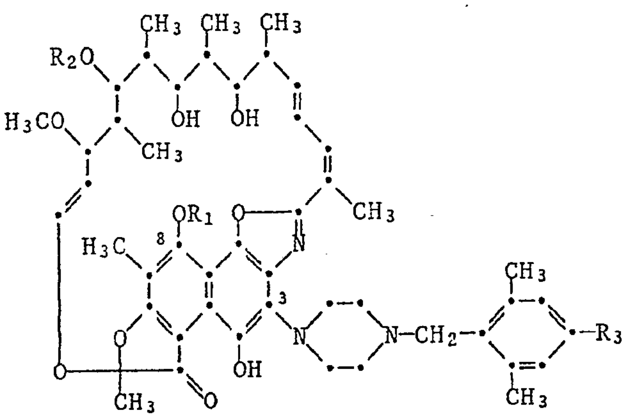

(I)

und ihre Salze, worin $R_1$ Wasserstoff oder Trialkylacetyl bedeutet, $R_2$ Wasserstoff oder Acetyl bedeutet und $R_3$ Alkyl bedeutet, welche wertvolle pharmakologische Eigenschaften besitzen.

EP 0 314 624 A1

## Beschreibung

### Substituierte Azacyclohexyl-Derivate

Gegenstand der Erfindung sind neue substituierte Azacyclohexyl-Derivate von Rifamycinen der Formel

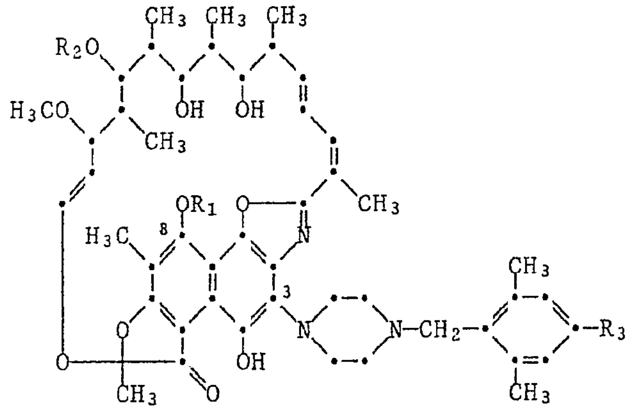

(I)

und ihre Salze, worin $R_1$ Wasserstoff oder Trialkylacetyl bedeutet, $R_2$ Wasserstoff oder Acetyl bedeutet und $R_3$ Alkyl bedeutet, ihre Herstellung und Verwendung sowie pharmazeutische Präparate und ihre Herstellung.

Die zugrundeliegende Numerierung des Ringsystems entspricht derjenigen, die z.B. im US Patent Nr. 4,005,077 angewandt wurde.

Die Verbindungen der Formel I besitzen mehrere Chiralitätszentren, dementsprechend umfasst die vorliegende Erfindung auch die entsprechenden optischen Isomeren, z.B. Diastereoisomeren.

Die Verbindungen der Formel I können als, insbesondere pharmazeutisch verwendbare, Salze vorliegen. Da die erfindungsgemässen Verbindungen basische Zentren aufweisen, können sie somit Säureadditionssalze bilden. Diese werden beispielsweise mit anorganischen Säuren, wie Mineralsäuren, z.B. Schwefelsäure, eine Phosphor- oder Halogenwasserstoffsäure, oder mit organischen Carbonsäuren, wie gegebenenfalls, z.B. durch Halogen, substituierte $C_1$-$C_4$-Alkancarbonsäuren, z.B. Essigsäure, wie gegebenenfalls ungesättigte Dicarbonsäuren, z.B. Oxal-, Malon-, Bernstein-, Malein-, Fumar-, Phthal-, oder Terephthalsäure, wie Hydroxycarbonsäuren, z.B. Glykol-, Milch-, Aepfel-, Wein- oder Citronensäure, wie Aminosäuren, z.B. Asparagin- oder Glutaminsäure, oder mit organischen Sulfonsäuren, wie gegebenenfalls, z.B. durch Halogen, substituierte $C_1$-$C_4$-Alkan- oder Arylsulfonsäuren, z.B. Methan-, Brombenzol- oder Toluolsulfonsäure, gebildet. Entsprechende Säureadditionssalze können auch mit dem zusätzlich vorhandenen basischen Zentrum gebildet werden. Ferner können die erfindungsgemässen Verbindungen mit einer aciden phenolischen Hydroxygruppe Salze mit Basen bilden, z.B. Alkalimetall-, wie Natrium-oder Kaliumsalze. Weiterhin können entsprechende innere Salze gebildet werden. Umfasst sind ferner für pharmazeutische Verwendungen nicht geeignete Salze, da diese beispielsweise für die Isolierung bzw. Reinigung erfindungsgemässer Verbindungen oder deren pharmazeutisch verwendbarer Salze eingesetzt werden können.

Trialkylacetyl bedeutet insbesondere Tri-$C_1$-$C_7$-alkylacetyl, bevorzugt Tri-$C_1$-$C_4$-alkyl-acetyl, wobei Alkyl jeweils die nachstehend angegebene Bedeutung hat. In erster Linie kommt Pivaloyl in Betracht.

Alkyl bedeutet insbesondere $C_1$-$C_7$-Alkyl und ist z.B. Methyl, Ethyl, Propyl, Isopropyl, n-Butyl, Isobutyl, sek.-Butyl, tert.-Butyl und umfasst ferner entsprechende Pentyl-, Hexyl-und Heptylreste. Bevorzugt ist $C_1$-$C_4$-Alkyl, in erster Linie jedoch Methyl.

Von Derivaten, die sich beispielsweise vom Rifamycin SV ableiten, ist bekannt, dass sie ausgeprägte antibiotische Eigenschaften besitzen und beispielsweise zur Behandlung von Tuberkulose eingesetzt werden können. Umso überraschender ist die experimentell verifizierte Feststellung, dass die Verbindungen der Formel I und ihre pharmazeutisch verwendbaren Salze in den üblichen pharmakologischen Testmodellen zur Prüfung keine entsprechende antibiotische Aktivität zeigen.

Ueberraschenderweise dagegen besitzen sie jedoch eine signifikante lipidsenkende Wirkung, die in Tierversuchen, vorzugsweise an Säugetieren, z.B. an Ratten, nachgewiesen werden kann. So kann die Senkung von "very low density"-, "low-density"- bzw. "high density"-Lipoproteinen (VLDL, LDL bzw. HDL) im Serum in zwei Testanordnungen, und zwar in genetisch hypercholesterolämischen männlichen Ratten (Anordnung A) und normolipämischen Ratten beiden Geschlechts (Anordnung B) gezeigt werden.

Albino-Ratten mit einem Körpergewicht von 180-240 g, die freien Zugang zu Standard-Rattenfutter und Trinkwasser haben, werden verwendet, und zwar Sprague Dawley Abkömmlinge des Stammes COBS Die Testverbindung wird in einer 3%igen Maisstärkelösung oral an Gruppen von 8 bis 10 Ratten täglich während 5 aufeinanderfolgenden Tagen verabreicht. Die Tiere werden zwei Stunden nach der letzten Gabe durch

Ausblutenlassen nach Herzstich unter Anästhesie mit Kohlendioxid getötet. Während 16 Stunden vor ihrem Tod erhalten die Tiere keine Nahrung mehr. Der Gesamtcholesterin-, Lipoprotein- und Triglyceridblutplasmaspiegel wird für jedes Versuchstier separat bestimmt. Zur Bestimmung der Lipoproteine werden in 1 ml mit Ethylendiamintetraessigsäure gegen Koagulation geschütztem Plasma die VLDL- und LDL-Fraktionen durch Hinzufügen von 200 Einheiten Heparin und von Manganchlorid bis zu einer Endkonzentration von 46 mMol/Liter ausgefällt und abzentrifugiert. Die überstehende Lösung wird mit dem restlichen Plasma vereinigt und der enzymatischen Bestimmung des Cholesterin- und Triglyceridgehaltes mit Hilfe der z.B. von Sigma Chemical Co. (St.Louis, MO, USA) gelieferten Testsysteme, zugeführt.

Die Prüfung auf antibiotische Wirkung erfolgt z.B. einerseits in vitro durch die Bestimmung der minimalen Hemmkonzentration MIC ("minimum inhibitory concentration") im konventionellen Plattentest. Als Mikroorganismen werden für den vorliegenden Zweck insbesondere Mycobacterium tuberculosis TB $H_{37}Rv$ und Staphylococcus aureus verwendet. Bei Verbindungen mit lipidsenkender Indikation wird eine antibiotische Wirksamkeit als nachteilig erachtet, da sie, insbesondere bei langfristiger Verabreichung, zur Bildung von gegen Antibiotika resistenten Stämmen von Mikroorganismen führen kann.

In den oben beschriebenen Testmethoden weisen die erfindungsgemässen Verbindungen bei wiederholter Verabreichung im Dosisbereich von etwa 1 bis etwa 10 mg/kg/Tag eine signifikante hypolipidämische Wirksamkeit auf. So kann z.B. gezeigt werden, dass, je nach Versuchsanordnung, die minimale effektive Dosis der erfindungsgemässen Verbindungen bei einmaliger Verabreichung bei etwa 1 bis etwa 3 mg/kg liegt, und durch eine wiederholte Verabreichung von täglich 10 mg/kg eine 75%ige Senkung der "LDL-Fraktion" erreicht werden kann. Ueberraschenderweise zeigen die erfindungsgemäss bereitgestellten Verbindungen praktisch keine antibiotische Wirksamkeit; die MIC für verschiedene pathogene Stämme von Staphylococcus aureus z.B. liegt über 130 µg/ml. Solche Werte sind etwa 1000-fach höher als Konzentrationen, die für einen entsprechenden Effekt normalerweise erforderlich sind.

Insbesondere dank ihrer LDL-senkenden Wirkung können die erfindungsgemässen Verbindungen z.B. als Hypolipidämika zur Behandlung von Hyperlipidämien, haputsächlich der Typen IIa und IIb, und Atherosclerose, z.B. bei Vorliegen von Hyperlipoproteinämie als Risikofaktor, verwendet werden.

Dementsprechend können die Verbindungen der Formel I und ihre pharmazeutisch verwendbaren Salze z.B. als Pharmazeutika, beispielsweise als Hypolipidämika zur Behandlung von Hyperlipidämien, hauptsächlich der Typen IIa und IIb, und von Arteriosklerose bei Vorliegen von Hyperlipoproteinamie als Risikofaktor, verwendet werden. Ein weiterer Gegenstand der Erfindung ist die Verwendung der erfindungsgemässen Verbindungen zur Herstellung von Arzneimitteln, insbesondere von Hypolipidämika und Antiarteriosklerotika, und zur therapeutischen und prophylaktischen Behandlung. Dabei ist auch die gewerbsmässige Herrichtung der Wirksubstanzen eingeschlossen.

Die Erfindung betrifft insbesondere Verbindungen der Formel I und ihre Salze, worin $R_1$ Pivaloyl und $R_3$ Methyl bedeuten.

Die Erfindung betrifft insbesondere die in den Beispielen beschriebenen neuen Verbindungen und ihre Herstellung.

Ebenfalls Gegenstand der Erfindung sind Verfahren zur Herstellung der erfindungsgemässen Verbindungen. Die Herstellung von Verbindungen der Formel I und ihrer Salze erfolgt in an sich bekannter Weise und ist z.B. dadurch gekennzeichnet, dass man eine Verbindung der Formel

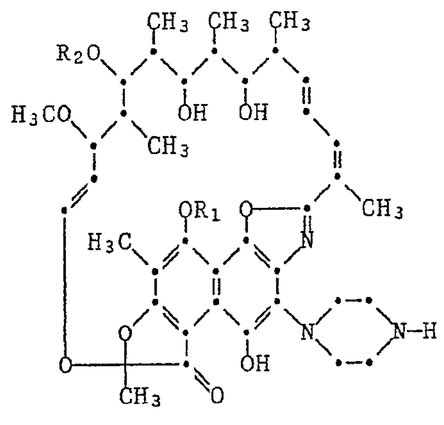

(IIa)

oder ein Salz davon mit einer Verbindung der Formel

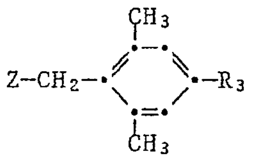

(IIb)

umsetzt, worin Z reaktionsfähiges verestertes Hydroxy bedeutet, oder,

b) zur Herstellung von Verbindungen der Formel I und ihrer Salze, worin $R_1$ Trialkylmethylcarbonyl bedeutet und $R_2$ Acetyl ist, eine Verbindung der Formel

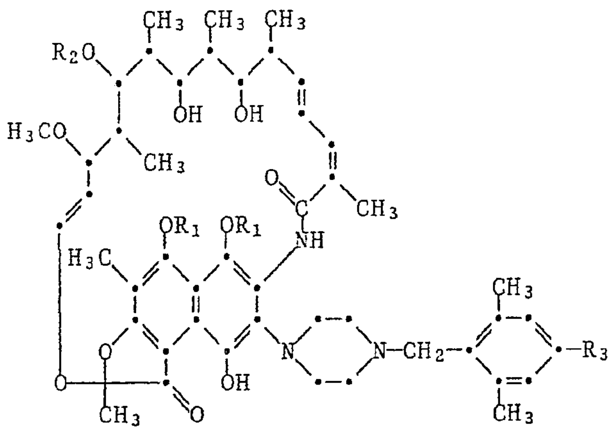

(III)

erwärmt, oder

c) eine Verbindung der Formel

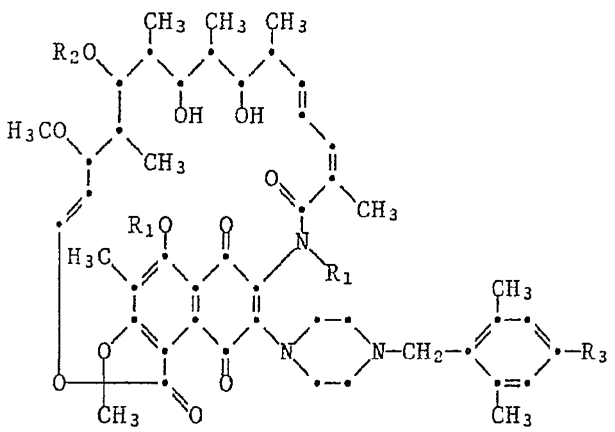

(IV)

erwärmt oder bestrahlt, und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt.

Salze der Ausgangsmaterialien der Formeln IIa, III und IV, die eine acide phenolische Hydroxygruppe aufweisen, sind entsprechende Salze mit Basen der vorstehend aufgeführten Art, während entsprechende Ausgangsverbindungen mit den basischen Zentren auch entsprechende Säureadditionssalze analog der Säureadditionssalze der Formel I bilden können.

Reaktionsfähiges verestertes Hydroxy ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfo-

nyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Brombenzol- oder p-Toluolsulfonyloxy.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs-oder Verdünnungsmittels oder eines Gemisches derselben, wobei man je nach Bedarf unter Kühlen, bei Raumtemperatur oder unter Erwärmen, z.B. in einem Temperaturbereich von etwa -80° bis zur Siedetemperatur des Reaktionsmediums, vorzugsweise von etwa -10° bis etwa +180° C, und, falls erforderlich in einem geschlossenen Gefäss, unter Druck, in einer Inertgasatmosphäre und/oder unter wasserfreien Bedingungen arbeitet.

Die Aufarbeitung des Reaktionsproduktes aus dem verfahrensgemäss erhältlichen Reaktionsgemisch erfolgt in an sich bekannter Weise, z.B. durch Verdünnen mit Wasser, und/oder gegebenenfalls durch Neutralisieren oder leichtes Ansäuern (bis etwa pH=3) mit einer wässrigen Säure, wie einer anorganischen oder organischen Säure, z.B. einer Mineralsäure oder, vorteilhafterweise, Zitronensäure, und Zugabe eines mit Wasser nichtmischbaren Lösungsmittels, wie eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes oder andere übliche Reinigungsmethoden in gereinigter Form erhalten werden kann.

Variante a):

Z bedeutet vorzugsweise Halogen, wie Chlor, Brom oder Iod, ferner Sulfonyloxy, wie Methan- oder p-Toluolsulfonyloxy.

Die Umsetzung erfolgt in an sich bekannter Weise, vorteilhaft in Gegenwart einer Base.

Als Basen kommen vorzugsweise nicht nucleophile tertiäre Amine in Frage, beispielsweise Triniederalkylamine, basische Heterocyclen und carbocyclische Amine, wie Ethyl-diisopropylamin, Triethylamin, Pyridin, 1,5-Diaza-bicyclo[4.3.0]non-5-en (DBN) sowie 1,8-Diaza-bicyclo[5.4.0]undec-7-en (DBU).

Variante b):

Die Behandlung von Verbindungen der Formel III erfolgt unter Erwärmen, z.B. in einem Temperaturbereich von etwa 50° bis 180° C, insbesondere von etwa 100° bis 170° C.

Das Ausgangsmaterial der Formel III kann man beispielsweise herstellen, indem man Rifamycin S oder 3-Halogen-, insbesondere 3-brom-, Rifamycin S mit einem Amin der Formel

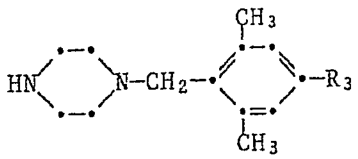

(IIIa)

umsetzt. Dabei arbeitet man insbesondere mit einem Ueberschuss an Amin der Formel IIIa beispielsweise in einem Temperaturbereich von etwa 0° bis etwa 100° C. Es bildet sich ein Gemisch aus der Chinon- und der Hydrochinonform. Dieses Gemisch kann mittels Reduktion, z.B. mit katalytischer Hydrierung, in das entsprechende Hydrochinon (Derivat von Rifamycin SV) übergeführt werden ($R_1$ = H). Durch Behandeln mit entsprechenden Acylierungsmitteln, z.B. mit einem Säureanhydrid, wie Pivaloylchlorid, in Gegenwart einer Base, wie Pyridin, kann man zu Verbindungen der Formel III gelangen, worin $R_1$ Trialkylacetyl bedeutet.

Variante c):

Die Umsetzung erfolgt insbesondere in einem organischen Lösungsmittel, beispielsweise einem Alkohol, wie Methanol, Ethanol oder Isopropanol, einem Keton, wie Aceton oder Methylethylketon einem chloriniertem Kohlenwasserstoff, wie Chloroform oder Trichlorethan, einem Ether, wie Diethylether, einer Base, wie Pyridin oder Triethylamin, oder einem Nitril, wie Acetonitril. Bevorzugte Lösungsmittel sind Isopropanol und Pyridin.

Falls die Temperatur zu niedrig ist, verläuft die Umsetzung sehr langsam, während bei zu hoher Temperatur grössere Anteile an unerwünschtem Nebenprodukt anfällt. Ein geeigneter Temperaturbereich liegt zwischen etwa 50° und etwa 90° C, bevorzugt etwa 75° C.

Die Bestrahlung wird in an sich bekannter Weise durchgeführt, beispielsweise unter Verwendung üblicher Bestrahlungsquellen, wie Mikrowellenbestrahlung.

Das resultierende Produkt kann z.B. durch Chromatographie und/oder durch Umkristallisation aus einem geeigneten Lösungsmittel, wie Petrolether, gereinigt bzw. isoliert werden.

Das Ausgangsmaterial der Formel IV kann in an sich bekannter Weise hergestellt werden, beispielsweise durch Behandeln einer Verbindung der Formel

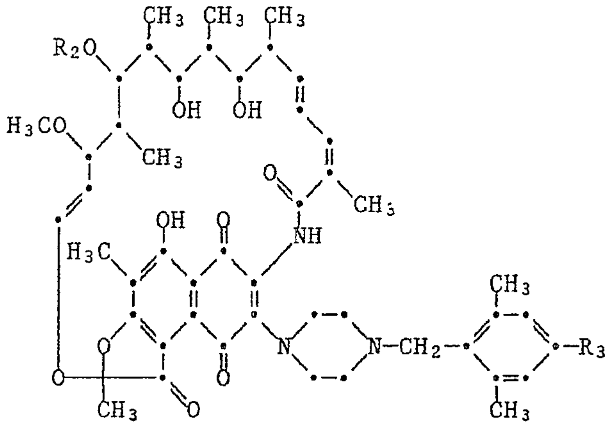

(IVa)

mit einem Acylierungsreagenz, welches die Trialkylacetylgruppe in Position 8 und 14 einführt.

Die Einführung der Trialkylacetylgruppe kann in an sich bekannter Weise erfolgen unter Verwendung eines geeigneten Acylierungsmittels, wobei mindestens zwei Aequivalente desselben eingesetzt werden. So kann man beispielsweise eine entsprechende Carbonsäure, falls erforderlich in Gegenwart eines geeigneten Kondensationsmittels, wie Dicyclohexylcarbodiimid, verwenden, vorzugsweise jedoch ein reaktionsfähiges Derivat einer derartigen Säure, wie ein Anhydrid, insbesondere ein gemischtes Anhydrid, z.B. mit einer anorganischen Säure, wie Halogenwasserstoffsäure, insbesondere Chlor- oder Bromwasserstoffsäure (das bedeutet, das entsprechende Säurehalogenid, z.B. - chlorid), oder mit einer organischen Säure, wie (Trifluor-)Essigsäure oder einem geeigneten Monoester oder Kohlensäure, oder alternativ ein symmetrisches Anhydrid oder ein inneres Anhydrid, z.B. das entsprechende Keten. Das Carbonsäurederivat, welches als Acylierungsmittel eingesetzt wird, wird vorzugsweise in Gegenwart eines basischen Mittels verwendet. Als geeignetes basisches Mittel kommt insbesondere eine nicht acylierbare organische Base, wie eine heteroaromatische Base, z.B. Pyridin, Collidin oder Chinolin, ein teritäres Amin, z.B. Triethylamin, N-Ethyl-piperidin, N-Methyl-Morpholin oder 1,4-Dimethyl-piperazin oder 1,5-Diazobicyclo[5.4.0]undec-5-en in Frage.

Die Acylierung wird üblicherweise in Gegenwart eines Lösungs- oder Verdünnungsmittels durchgeführt, wobei auch ein Ueberschuss des Acylierungsmittels oder der Base, z.B. Pyridin, zusammen mit einem Acylierungsmittel verwendet wird. Andere Lösungmittel, die beispielsweise ebenfalls mit einer Base vermischt sein können, sind z.B. nicht acylierbare organische Lösungsmittel, wie Kohlenwasserstoff, z.B. Pentan, Hexan oder Cyclohexan, halogenierte Kohlenwasserstoffe, z.B. Methylenchlorid oder Chloroform, Ether, z.B. Diethylether, Ethylenglykoldimethylether, Tetrahydrofuran oder Dioxan, Säureester, wie Ethylacetat, Säureamide, z.B. Acetamid oder Dimethylformamid.

Die Umsetzung wird üblicherweise bei Raumtemperatur oder bei leicht erhöhter Temperatur durchgeführt, z.b. bis etwa 70°C, wobei die Reaktion erforderlichenfalls unter Inertgasatmosphäre durchgeführt wird. Die Acylierungsbedingungen, insbesondere die Menge an Acylierungsmittel, das Reaktionsmedium, die Temperatur und die Reaktionszeit sollten so gewählt werden, dass beide Acylgruppen eingeführt werden, wobei vorzugsweise die in den Beispielen detailliert beschriebene Methodik anzuwenden ist. Der Reaktionsverlauf kann vorteilhaft mit Hilfe üblicher analytischer Methoden, insbesondere mit Dünnschichtchromatographie, verfolgt werden.

Das Ausgangsmaterial der Formel IVa ist bekannt bzw. kann in an sich bekannter Weise hergestellt werden, wobei insbesondere auf die PCT-Anmeldung mit der Publikationsnummer WO 87/02361 Bezug zu nehmen ist.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I oder Salz davon kann in an sich bekannter Weise in eine andere Verbindung der Formel I übergeführt werden.

Verbindungen der Formel I, worin $R_1$ Wasserstoff ist, können in an sich bekannter Weise acyliert werden, beispielsweise durch Umsetzung mit der entsprechenden Carbonsäure oder einem reaktionsfähigen Derivat davon. Derartige reaktionsfähige Derivate sind beispielsweise Anhydride, inklusive gemischte Anhydride, wie ein Säurehalogenid, z.B. -chlorid, oder Anhydride mit einem Ameisensäureester, aktivierte Carbonsäureester, wie Cyanmethyl-, (4-)Nitrophenyl-, Polyhalogenphenyl-, z.B. Pentachlorphenyl-, -ester. Die Umsetzung mit der Carbonsäure oder einem Salz davon erfolgt unter wasserabspaltenden Bedingungen, z.B. unter aceotroper Entfernung des Reaktionswassers, oder durch Behandeln mit einem geeigneten Kondensationsmittel, z.B. N,N′-Dicyclohexy-carbodiimid. Die Umsetzung mit einem reaktionsfähigen Säurederivat wird vorteilhaft in Gegenwart einer Base durchgeführt. Entsprechend kann durch Behandeln mit einem entsprechenden Acetylierungsmittel der Acetylrest $R_2$ in Verbindungen der Formel I, worin $R_2$ Wasserstoff ist, eingeführt werden.

Durch Behandeln mit starken Basen, wie Alkalimetallhydroxiden, können der Acetylrest $R_2$ und der Acylrest

$R_1$ durch Wasserstoff ersetzt werden. Der Acylrest $R_1$ kann auch in Gegenwart des Acetylrests $R_2$ selektiv abgespalten werden, beispielsweise durch Behandeln mit einem Fluorid, wie Alkalimetall-, z.B. Natrium- oder Cäsiumfluorid, oder einem Ammoniumfluorid, z.B. Tetrabutylammoniumfluorid.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung in freier Form und in Form ihrer Salze sind im Vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, insbesondere neue Verbindungen der Formel III, ihre Verwendung und Verfahren zu ihrer Herstellung bilden ebenfalls einen Gegenstand der Erfindung, wobei die Variablen $R_1$, $R_2$ und $R_3$ die für die jeweils bevorzugten Verbindungsgruppen der Formel I davon angegebenen Bedeutungen haben.

Die vorliegende Erfindung umfasst auch die Verwendung der Verbindungen der Formel I und ihrer Salze allein oder zusammen mit Hilfsstoffen, sowie auch in Kombination mit anderen Wirkstoffen, als Mittel zur therapeutischen, und zwar sowohl kurativen wie auch präventiven Behandlung von Krankheiten oder krankhaften Zuständen, die z.B. durch einen erhöhten Gehalt an Cholesterin und/oder Triglyceriden im Blut, insbesondere im Blutserum, angezeigt oder verursacht werden. Die erfindungsgemässen Wirkstoffe werden in therapeutisch wirksamen Mengen, vorzugsweise in Form von pharmazeutischen Zusammensetzungen zusammen mit konventionellen pharmazeutischen Trägermaterialien und/oder Hilfsstoffen an den behand lungsbedürftigen Warmblüter, in erster Linie Menschen, verabreicht. Dabei werden z.B. an Warmblüter je nach Spezies, Körpergewicht, Alter und individuellem Zustand, tägliche Dosen entsprechend etwa 1 bis etwa 100, insbesondere etwa 3 bis etwa 50 mg pro kg Körpergewicht, die in schweren Fällen überschritten werden können, verabreicht. Sinngemäss umfasst die Erfindung auch die entsprechende Methode zur medizinischen Behandlung.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen ferner rektalen, und parenteralen Verabreichung an Warmblüter(n), wobei der pharmakologische Wirkstoff allein oder zusammen mit einem pharmazeutisch anwendbaren Trägermaterial enthalten ist.

Die neuen pharmazeutischen Präparate enthalten z.B. von etwa 10 % bis etwa 80%, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-,

Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferne Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxmethylstärke, quervernetzes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs hängt von der Warmblüter-Spezis, dem Alter und dem individuellen Zustand sowie der Applikationsweise ab. Im Normalfall ist für einen etwa 75 kg schweren Warmblüter bei oraler Applikation eine ungefähre Tagesdosis von etwa 150 mg bis etwa 1500 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken.

Beispiel 1:

30 g 1,8-Di-O-pivaloyl-3-[4-(2,4,6-trimethylbenzyl)-piperazin-1-yl]-rifamycin SV werden in der Wärme in 1000 ml 2-Methoxyäthanol gelöst und unter Stickstoff während 5 Stunden unter Rückfluss gekocht. Dann dampft man das Lösungsmittel im Vakuum ein und kristallisiert den Rückstand zweimal aus Methanol. Man erhält das 8-O-Pivaloyl-1-desoxy-15-desoxo-1,15-oxy-3-[4-(2,4,6-trimethylbenzyl)-piperazin-1-yl]-rifamycin der Formel I, worin $R_1$ Pivaloyl bedeutet, $R_2$ Acetyl bedeutet und $R_3$ Methyl bedeutet. Schmelzpunkt 160-165°C. $C_{56}H_{73}N_3O_{12}$; M = 979, gefunden (MS): 979; $^1$H-NMR (360 MHz, $CDCl_3$, TMS): 1,49 (s, 9H, Pivaloyl an 0-8).

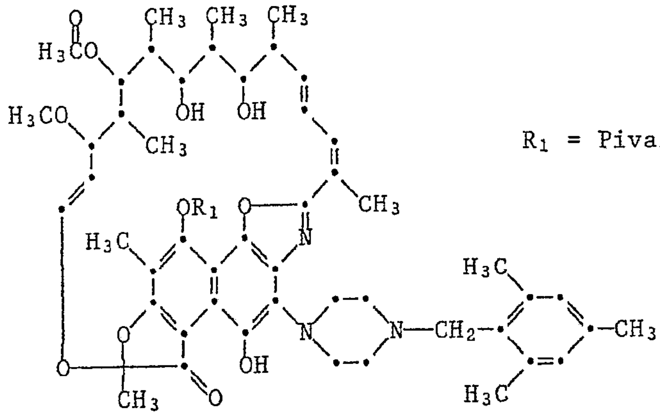

$R_1$ = Pivaloyl

Das Ausgangsmaterial kann wie folgt hergestellt werden:
Ein Gemisch aus 5 g 3-[4-(2,4,6-Trimethylbenzyl)-piperazin-1-yl]-rifamycin SV, 50 ml trockenem Pyridin und 4,5 ml Pivaloylchlorid wird während 30 Minuten auf 50°C gehalten. Anschliessend verdampft man das Lösungsmittel im Vakuum. Der ölige Rückstand wird in Essigester gelöst und mit 2N-Salzsäure, mit Pufferlösung pH = 7 und mit Kochsalzlösung gewaschen. Nach dem Trocknen über Natriumsulfat und Eindampfen wird der gelbe Rückstand aus Aether/Hexan kristallisiert. Man erhält so das 1,8-Di-O-pivaloyl--3-[4-(2,4,6-trimethylbenzyl)-piperazin-1-yl]-rifamycin SV vom Schmelzpunkt 203-204°C. $C_{61}H_{83}N_3O_{16}$; MG: 1081 (gefunden, MS).

Beispiel 2:
Man versetzt eine Lösung von 10,7 g des Zielprodukts von Beispiel 1 in 200 ml Tetrahydrofuran bei Raumtemperatur unter Rühren portionsweise solange mit festem Tetrabutylammoniumfluorid-trihydrat, bis die anfangs rote Farbe der Lösung nach gelb umgeschlagen ist. Danach setzt man Wasser zu, säuert mit Zitronensäure an und nimmt das Reaktionsprodukt in Essigester auf. Nach Waschen des Essigesterextraktes mit Wasser und Kochsalzlösung trocknet man über Natriumsulfat und dampft im Vakuum ein. Der Rückstand wird aus Aether kristallisiert. Man erhält so das 1-Desoxy-15-desoxo-1,15-oxy-3-[4-(2,4,6-trimethylbenzyl)-piperazin-1-yl]-rifamycin der Formel I, worin $R_1$ Wasserstoff ist, $R_2$ Acetyl bedeutet und $R_3$ Methyl bedeutet, in langen zitronengelben Prismen, die aus Methanol/Wasser umkristallisiert werden. Schmelzpunkt 175°C. $C_{51}H_{65}N_3O_{11}$; MG: 895 (gefunden, MS,FD). UV-Spektrum in 0,01N alkoholischer HCl, Maxima in nm/ε: 241/37240; 298/23000; 330 (Schulter); 437/9920.

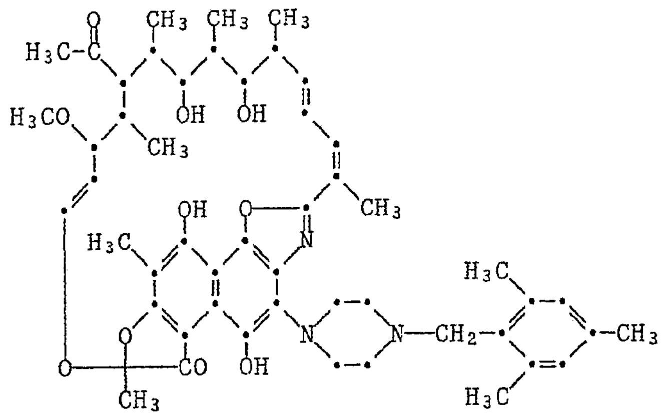

Beispiel 3:
Eine Lösung des Reaktionsproduktes von Beispiel 2 in Dioxan-1N Natronlauge (1:1) wird bei Raumtemperatur 21 Stunden stehen gelassen. Danach verdünnt man mit Wasser, säuert mit Zitronensäure an, nimmt das Reaktionsprodukt mit Essigester auf und kristallisiert aus Essigester/Aether. Man erhält so das 25-Desacetyl-3-[4-(2,4,6-trimethylbenzyl)-piperazin-1-yl]-1-desoxy-15-desoxo-1,15-oxy-rifamycin der Formel

I, worin $R_1$ Wasserstoff ist, $R_2$ Wasserstoff ist und $R_3$ Methyl bedeutet, als gelbe Kristalle vom Schmelzpunkt 190-195°C (Zersetzung). $C_{49}H_{63}N_3O_{10}$; MG: 853 (gefunden, MS,FD).

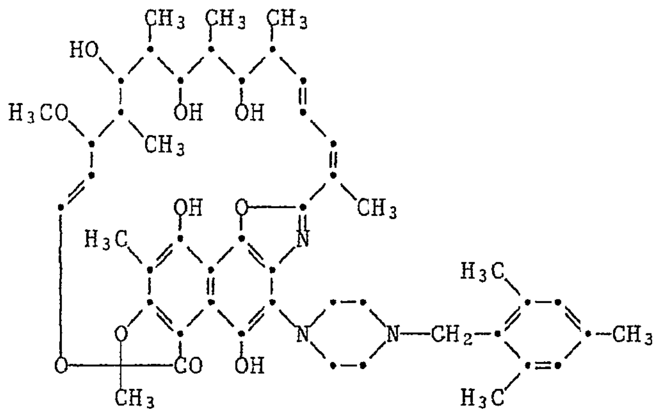

Beispiel 4:

Eine Lösung des in Beispiel 3 beschriebenen Zielproduktes in Pyridin wird mit 10 Aequivalenten Pivaloylchlorid versetzt und bei Raumtemperatur so lange stehen gelassen, bis bei Dünnschicht-chromatographischer Kontrolle im Reaktionsprodukt kein Ausgangsmaterial mehr nachgewiesen werden kann. Nun dampft man im Hochvakuum rasch zur Trockne ein, nimmt den Rückstand in Methylenchlorid auf, wäscht den Methylenchloridextrakt nacheinander mit Zitronensäurelösung, Pufferlösung von pH = 7 und gesättigter Kochsalzlösung. Nach dem Trocknen und Eindampfen des Methylenchloridextraktes bleibt ein roter Rückstand, das 25-Desacetyl-8-O-pivaloyl-3-[4-(2,4,6-trimethylbenzyl)-piperazin-1-yl]-1-desoxy-15-desoxo-1,15-oxy-rifamycin der Formel I, worin $R_1$ Pivaloyl bedeutet, $R_2$ Wasserstoff ist und $R_3$ Methyl bedeutet. $C_{54}H_{71}N_3O_{11}$; MG: 937 (gefunden, MS,FD). $^1$H-NMR ($CDCl_3$, 360 MHz): Signal der Pivaloylgruppe bei 1.41 (s, 9H, $CH_3C$).

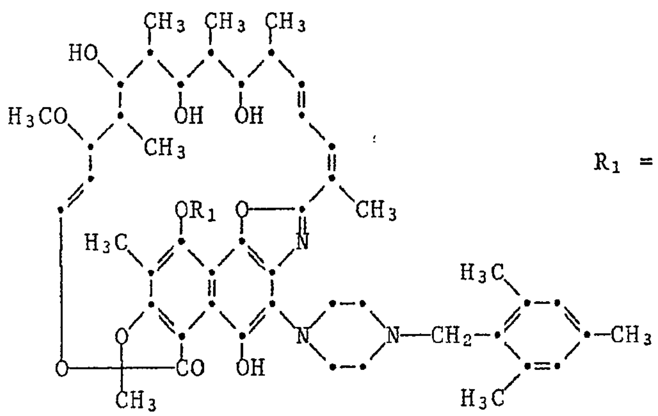

$R_1$ = Pivaloyl

Beispiel 5:

4 g 8-O,N-Dipivaloyl-3-[4-(2,4,6-trimethylbenzyl)-piperazin-1-yl]-rifamycin S (hergestellt gemäss WO 87/02361, Beispiel 1) werden lichtgeschützt in 70 ml Isopropanol im Bombenrohr 4 Stunden auf 100° erhitzt. Anschliessend wird das Lösungsmittel im Vakuum abgezogen und der dunkelrote Rückstand an 600 g Kieselgel (Merck) mit Petrolether/Essigester (3:2) chromatographiert. Die in den beiden ersten Zonen enthaltenen Substanzen werden verworfen. Die darauf folgende rote Fraktion enthält das Zielprodukt von Beispiel 1. Weiteres auf der Chromatographiesäule befindliches Zielprodukt wird mit Essigester/Methanol (9:1) eluiert und zur weiteren Reinigung nochmals chormatographiert. Nach Eindampfen der Eluate erhält man das in Beispiel 1 charakterisierte 8-O-Pivaloyl-1-desoxy-15-desoxo-1,15-oxy-3-[4,2,6-trimethylbenzyl)-piperazin-1-yl]-rifamycin.

Beispiel 6:
Kapseln, enthaltend 250 mg als Wirkstoff z.B. die Verbindung der Formel I, worin $R_1$ Pivaloyl bedeutet, $R_2$ Acetyl bedeutet und $R_3$ 2,4,6-Trimethylphenyl bedeutet, können wie folgt hergestellt werden:

Zusammensetzung (für 1000 Kapseln):

| | |
|---|---|
| Wirkstoff | 250,0 g |
| Maisstärke | 50,0 g |
| Polyvinylpyrrolidon | 15,0 g |
| Magnesiumstearat | 5,0 g |
| Ethanol | q.s. |

Der Wirkstoff und die Maisstärke werden vermischt und mit einer Lösung des Polyvinylpyrrolidons in 50 g Ethanol befeuchtet. Die feuchte Masse wird durch ein Sieb mit einer Maschenweite von 3 mm gepresst und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse O abgefüllt.

In analoger Weise kann man auch die übrigen, gemäss Beispielen hergestellten Verbindungen als Wirkungskomponente verwenden.

Beispiel 7:
250 g eines Wirkstoffes gemäss einem der Beispiele 1 bis 5 und 1750 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2 g gegossen. Sie enthalten je 250 mg Wirkstoff.

**Patentansprüche**

1. Verbindungen der Formel

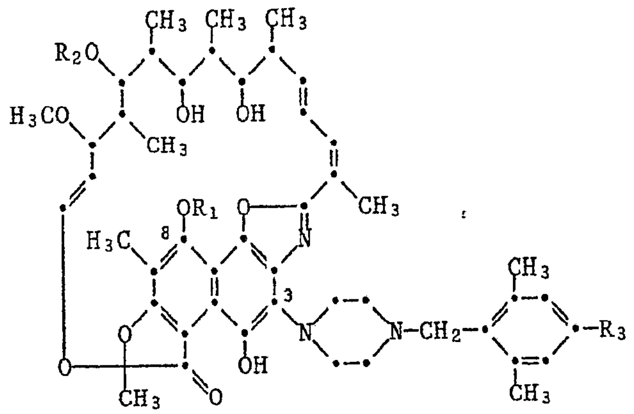

(I)

und ihre Salze, worin $R_1$ Wasserstoff oder Trialkylacetyl bedeutet, $R_2$ Wasserstoff oder Acetyl bedeutet und $R_3$ Alkyl bedeutet.

2. Verbindung gemäss Anspruch 1 der Formel I oder ein Salz davon, worin $R_1$ Tri-$C_1$-$C_7$-alkyl-acetyl, insbesondere Tri-$C_1$-$C_4$-alkyl-acetyl, bedeutet und $R_3$ $C_1$-$C_7$-Alkyl, insbesondere $C_1$-$C_4$-Alkyl, bedeutet.

3. Verbindung gemäss Anspruch 1 oder 2 der Formel I oder ein Salz davon, worin $R_1$ Pivaloyl bedeutet.

4. Verbindung gemäss einem der Ansprüche 1-3 der Formel I oder ein Salz davon, worin $R_3$ Methyl bedeutet.

5. Verbindung gemäss Anspruch 1 der Formel I oder ein Salz davon, worin $R_1$ Pivaloyl bedeutet und $R_3$ Methyl bedeutet.

6. Verbindung gemäss Anspruch 1 der Formel I oder ein Salz davon, worin $R_1$ Pivaloyl bedeutet, $R_2$ Acetyl bedeutet und $R_3$ Methyl bedeutet.

7. Verbindung gemäss Anspruch 1 der Formel I oder ein Salz davon, worin $R_1$ Wasserstoff ist, $R_2$ Acetyl bedeutet und $R_3$ Methyl bedeutet.

8. Verbindung gemäss Anspruch 1 der Formel I oder ein Salz davon, worin $R_1$ und $R_2$ jeweils Wasserstoff bedeuten und $R_3$ Methyl bedeutet.

9. Verbindung gemäss Anspruch 1 der Formel I oder ein Salz davon, worin $R_1$ Pivaloyl bedeutet, $R_2$ Wasserstoff ist und $R_3$ Methyl bedeutet.

10. Verbindung gemäss einem der Ansprüche 1-9 in Form eines pharmazeutisch verwendbaren Salzes.

11. Verbindung gemäss einem der Ansprüche 1-10 zur therapeutischen Behandlung des menschlichen oder tierischen Körpers.

12. Verbindung gemäss einem der Ansprüche 1-10 zur Anwendung als Hypolipidämikum und Antisklerotikum.

13. Pharmazeutische Präparate enthaltend als Wirkstoff eine Verbindung gemäss einem der Ansprüche 1-10 in freier Form oder in Form eines pharmazeutisch verwendbaren Salzes.

14. Verwendung einer Verbindung gemäss einem der Ansprüche 1-9 in freier Form oder in Form eines pharmazeutisch verwendbaren Salzes davon zur Herstellung von pharmazeutischen Präparaten zur Behandlung von Hyperlipidämie und Artherosclerose.

15. Verfahren zur Herstellung einer Verbindung gemäss einem der Ansprüche 1-10, dadurch gekennzeichnet, dass man eine Verbindung der Formel

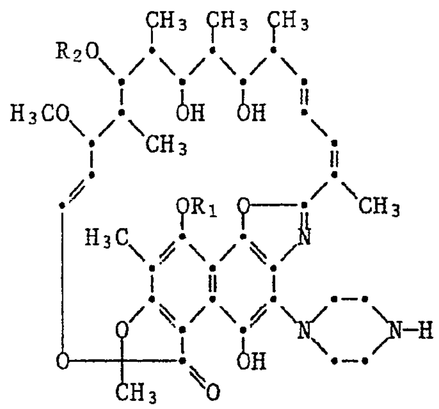

(IIa)

oder ein Salz davon mit einer Verbindung der Formel

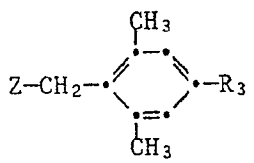

(IIb)

umsetzt, worin Z reaktionsfähiges verestertes Hydroxy bedeutet, oder,

b) zur Herstellung von Verbindungen der Formel I und ihrer Salze, worin $R_1$ Trialkylmethylcarbonyl bedeutet und $R_2$ Acetyl ist, eine Verbindung der Formel

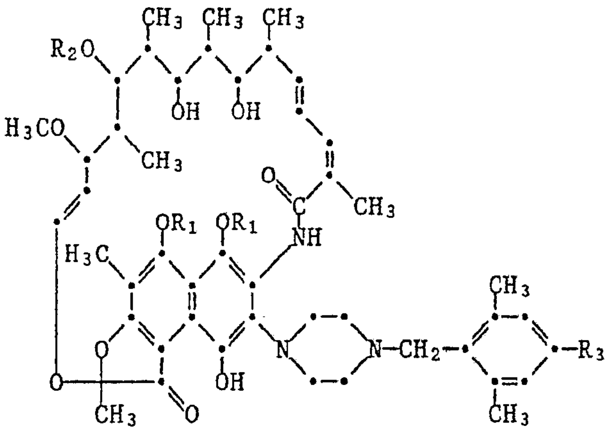

(III)

erwärmt, oder

c) eine Verbindung der Formel

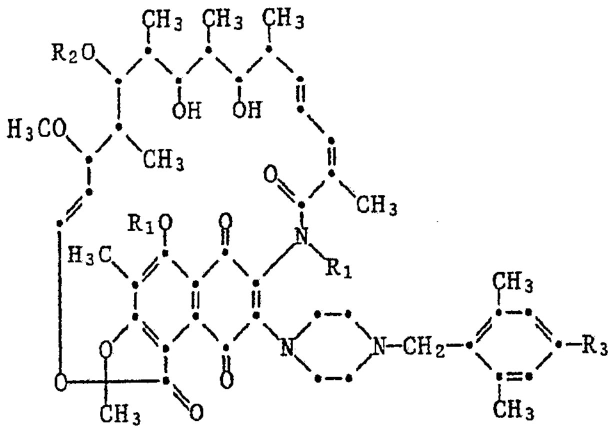

(IV)

erwärmt oder bestrahlt, und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt.

**Patentansprüche für folgende Vertragsstaaten: ES, GR**

1. Verfahren zur Herstellung von Verbindungen der Formel

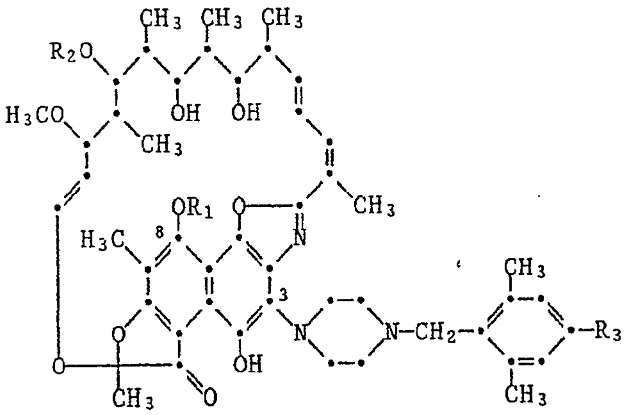

(I)

und ihrer Salze, worin $R_1$ Wasserstoff oder Trialkylacetyl bedeutet, $R_2$ Wasserstoff oder Acetyl bedeutet und $R_3$ Alkyl bedeutet, dadurch gekennzeichnet, dass man eine Verbindung der Formel

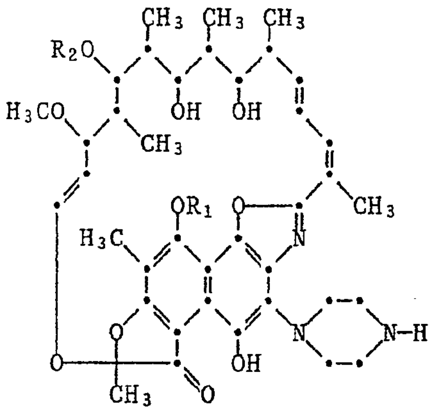

(IIa)

oder ein Salz davon mit einer Verbindung der Formel

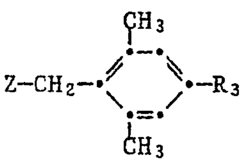

(IIb)

umsetzt, worin Z reaktionsfähiges verestertes Hydroxy bedeutet, oder,

b) zur Herstellung von Verbindungen der Formel I und ihrer Salze, worin $R_1$ Trialkylmethylcarbonyl bedeutet und $R_2$ Acetyl ist, eine Verbindung der Formel

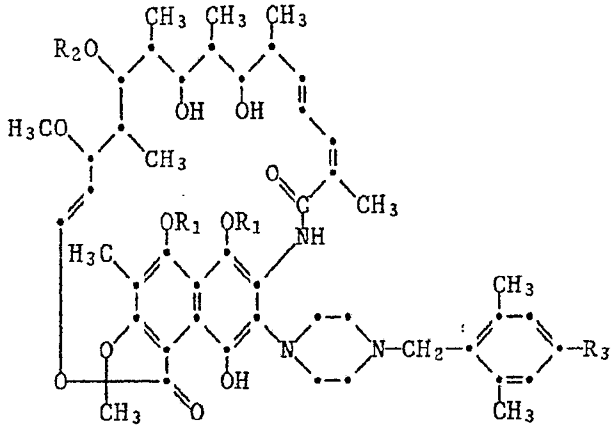

(III)

erwärmt, oder

c) eine Verbindung der Formel

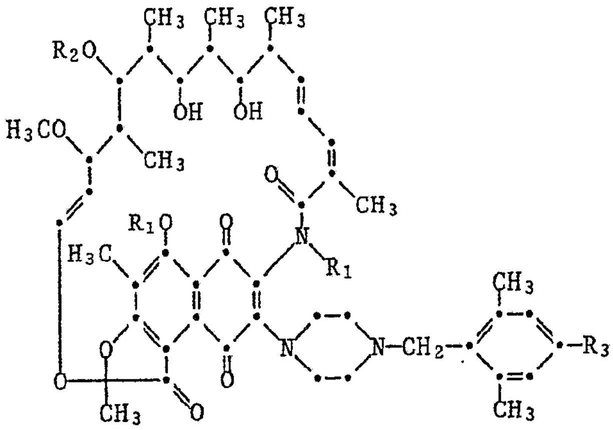

(IV)

erwärmt oder bestrahlt, und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt.

2. Verfahren gemäss Anspruch 1 zur Herstellung von Verbindungen der Formel I und ihrer Salze, worin $R_1$ Tri-$C_1$-$C_7$-alkyl-acetyl, insbesondere Tri-$C_1$-$C_4$-alkyl-acetyl, bedeutet und $R_3$ $C_1$-$C_7$-Alkyl, insbesondere $C_1$-$C_4$-Alkyl, bedeutet.

3. Verfahren gemäss Anspruch 1 zur Herstellung von Verbindungen der Formel I und ihrer Salze, worin $R_1$ Pivaloyl bedeutet.

4. Verfahren gemäss Anspruch 1 zur Herstellung von Verbindungen der Formel I und ihrer Salze, worin $R_3$ Methyl bedeutet.

5. Verfahren gemäss Anspruch 1 zur Herstellung von Verbindungen der Formel I und ihrer Salze, worin $R_1$ Pivaloyl und $R_3$ Methyl bedeuten.

6. Verfahren gemäss Anspruch 1 zur Herstellung von Verbindungen der Formel I und ihrer Salze, worin $R_1$ Pivaloyl bedeutet, $R_2$ Acetyl bedeutet und $R_3$ Methyl bedeutet.

7. Verfahren gemäss Anspruch 1 zur Herstellung von Verbindungen der Formel I und ihrer Salze, worin $R_1$ Wasserstoff ist, $R_2$ Acetyl bedeutet und $R_3$ Methyl bedeutet.

8. Verfahren gemäss Anspruch 1 zur Herstellung von Verbindungen der Formel I und ihrer Salze, worin $R_1$ und $R_2$ jeweils Wasserstoff bedeuten und $R_3$ Methyl bedeutet.

9. Verfahren gemäss Anspruch 1 zur Herstellung von Verbindungen der Formel I und ihrer Salze, worin $R_1$ Pivaloyl bedeutet, $R_2$ Wasserstoff ist und $R_3$ Methyl bedeutet.

10. Verfahren gemäss Anspruch 1 zur Herstellung von Verbindungen der Formel I in Form eines pharmazeutisch verwendbaren Salzes.

11. Verfahren zur Herstellung von pharmazeutischen Präparaten, dadurch gekennzeichnet, dass man eine Verbindung gemäss einem der Ansprüche 1-9 oder ein pharmazeutisch verwendbares Salz davon

gegebenenfalls unter Verwendung üblicher Hilfs- und Trägerstoffe zu pharmazeutischen Präparaten verarbeitet.

12. Verwendung einer Verbindung gemäss einem der Ansprüche 1-9 in freier Form oder in Form eines pharmazeutisch verwendbaren Salzes davon zur Herstellung von pharmazeutischen Präparaten zur Behandlung von Hyperlipidämie und Artherosklerose.

Europäisches Patentamt

# EUROPÄISCHER RECHERCHENBERICHT

Nummer der Anmeldung

EP 88 81 0721

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | WO-A-8 702 361 (CIBA-GEIGY)<br>* Ansprüche 1,8 *<br>--- | 1,13 | C 07 D 498/18<br>A 61 K 31/495//<br>(C 07 D 498/18<br>C 07 D 323:00<br>C 07 D 307:00<br>C 07 D 263:00 ) |
| Y | CHEMICAL PHARMACEUTICAL BULLETIN, Band 33, Nr. 5, 1985, Seite 2133-2136, Tokyo, JP; M. TAGUCHI et al.: "Chemical modification of the amide group of rifamycin S"<br>* Formel 8 *<br>----- | 1 | |

| RECHERCHIERTE SACHGEBIETE (Int. Cl.4) |
|---|
| C 07 D 498/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 17-01-1989 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)